# EUROPEAN PATENT APPLICATION

(11) **EP 4 616 865 A1**
(43) Date of publication of application: **17.09.2025**
(21) Application number: 24163309.8
(22) Date of filing: 13.03.2024
(51) Int. Cl.: A61K 38/18, A61P 27/02

(54) **BDNF FOR THE TREATMENT OF RHODOPSIN-MEDIATED RETINITIS PIGMENTOSA**

(71) Applicant: Dompe' Farmaceutici SpA, 20122 Milano (IT)
(72) Inventor: ARAMINI, Andrea, 67100 L'Aquila (IT); SIRICO, Anna, 67100 L'Aquila (IT); BRANDOLINI, Laura, 67100 L'Aquila (IT); NOVELLI, Rubina, 20122 Milan (IT)
(74) Representative: Dompé farmaceutici Spa

(57) **Abstract**

The present invention relates to brain derived nerve factor (BDNF) for use in the treatment of rhodopsin-mediated retinitis pigmentosa in a subject.

## Description

### FIELD OF THE INVENTION

The present invention relates to the treatment of a rhodopsin-mediated retinitis pigmentosa.

### STATE OF THE ART

Retinitis pigmentosa encompasses a heterogeneous group of inherited retinal dystrophies characterized by the degeneration of rod and cone photoreceptors, affecting approximately 1 in 4000 people worldwide.

The disease is characterized by the initial progressive degeneration and death of rod photoreceptors, resulting in a loss of night vision (nyctalopia) and a progressive constriction of the visual field. At a later stage, rod cells depletion induces secondary degeneration of cones, possibly through a bystander effect, eventually causing complete blindness.

Retinitis pigmentosa is genetically heterogeneous, showing autosomal recessive, autosomal dominant, and X-linked inheritance patterns. Causative mutations for retinitis pigmentosa have been found to date in more than 80 genes (Verbakel et al, Progress in Retinal and Eye Research 2018, 66: 157-186).

Rhodopsin (RHO) is a G-protein coupled receptor localized in the outer segment of rod cells in the retina, that is responsible for visual phototransduction in these cells.

Mutations in the rhodopsin-encoding gene were the first molecular defects identified in retinitis pigmentosa and account for about 30% of autosomal dominant RP (adRP) cases of retinitis pigmentosa in United States and 8-10% of all RP (Hartong et al, Lancet 2006, 368, 1795-1809; Athanasiou et al, Prog Retin Eye Res 2018, 62:1-23). To date more than 150 mutations have been associated with an adRP phenotype, with the P23H mutation being the most frequent (Yan et al, Molecular Therapy: Nucleic Acids 2023, Vol. 33, pages 750-761).

Rhodopsin mutations associated with adRP have been classified into six categories based on their biochemical and cellular defects, and most of them belong to Class I or Class II. Class I mutations, located exclusively at the C-terminal of the protein, can fold properly and form a functional photoreceptor but are not correctly transported to the outer segment. Class II mutations, which occur in the intradiscal, transmembrane, and cytoplasmic domains of the protein, lead to misfolded protein that cannot reconstitute with 11-cis-retinaldehyde to form functional rhodopsin and is retained in the endoplasmic reticulum (Athanasiou et al, Prog Retin Eye Res 2018, 62:1-23; Xiao et al, Eye 2019 33:592-599). The P23H mutation belongs to class II mutations.

Although uncommon, a few rhodopsin mutations have also been identified that are associated with autosomal recessive RP (arRP).

Currently, there is only one approved therapy for retinitis pigmentosa, which is directed to a very limited number of patients. Voretigene neparvovec (Luxturna^{®}) is a recombinant adeno-associated virus vector-based gene therapy, that delivers a functioning copy of the human retinal pigment epithelium-specific 65 kDa protein (RPE65) gene into retinal cells of patients that have RPE65 mutation-associated retinitis pigmentosa. However, this patient population represents 0.3-1% of total RP cases. There is currently no standard treatment for other patients, that only rely in supportive care such as vitamin supplements, protection from sunlight and visual aids.

It is therefore strongly felt the need to develop novel therapies for the treatment of retinitis pigmentosa, especially for the most frequent form of this retinal dystrophy such as rhodopsin-mediated retinitis pigmentosa.

Brain-derived neurotrophic factor (BDNF) is a neurotrophic factor that support differentiation, maturation, and survival of neurons in the nervous system and shows a neuroprotective effect under adverse conditions, such as glutamatergic stimulation, cerebral ischemia, hypoglycemia, and neurotoxicity (Bathina et al, Arch Med Sci. 2015; 11(6): 1164-1178.).

In view of its pro-survival and neuroprotective activity, intravitreal administration of BDNF has been tested in models of retinal degeneration. However, in a study of the effect of BDNF on different models of inherited RP, a modest partial rescue was observed only in one out of seven models (LaVail MM et al, Proc Natl Acad Sci USA 1992,89:11249-11253; LaVail MM et al, Invest Ophthalmol Vis Sci 1998, 39:592-602). These data have also been confirmed by a subsequent study (Ogilvie JM et al, Experimental Neurology 2000, 161, 676-685). Similarly, in a model of cone-rod dystrophy, BDNF failed to prevent photoreceptor degeneration (Chong et al, Invest Ophthalmol Vis Sci. 1999, 40(6):1298-305.).

In the above studies, it has also been hypothesized that different pathological mechanisms associated with different genetic alteration in retinitis pigmentosa may result in a specific susceptibility to the activity of pro-survival protective factors (LaVail MM et al, Invest Ophthalmol Vis Sci 1998, 39:592-602.

### SUMMARY OF THE INVENTION

As it will described in the experimental section, the present inventors have now demonstrated that the intravitreal administration of BDNF protects photoreceptors from degeneration in rhodopsin-mediated retinitis pigmentosa and rescues, in a dose dependent way, retinal function and morphology, inducing significant recovery of visual ability.

Accordingly, a first object of the invention is BDNF for use in the treatment of rhodopsin-mediated retinitis pigmentosa in a subject.

A further object of the invention is an ophthalmic composition comprising BDNF and at least one ophthalmically acceptable excipient, for use in the treatment of rhodopsin-mediated retinitis pigmentosa in a subject.

### BRIEF DESCRIPTION OF THE FIGURES

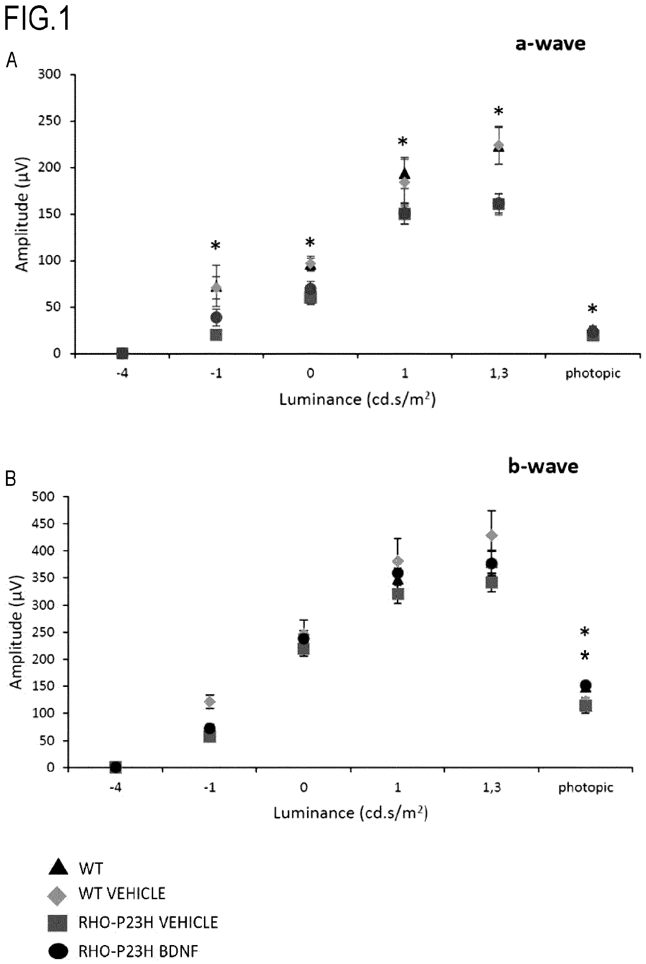

**Figure 1** shows ERG a-wave amplitude (panel a) and b-wave (panel b) amplitude responses recorded at P30 in WT untreated mice (WT), WT mice treated with vehicle (WT VEHICLE), RHO-P23H mice treated with vehicle (RHO-P23H VEHICLE), and RHO-P23H mice treated with a single injection of rhBDNF (RHO-P23H BDNF), according to Example 1. Error bars represent SEM; statistical significance is indicated with asterisks (unpaired Student's t test, Vehicle vs WT (six-pointed asterisks), and rhBDNF vs Vehicle (five-pointed asterisks)) (*p ≤ 0.05; *p ≤ 0.05).

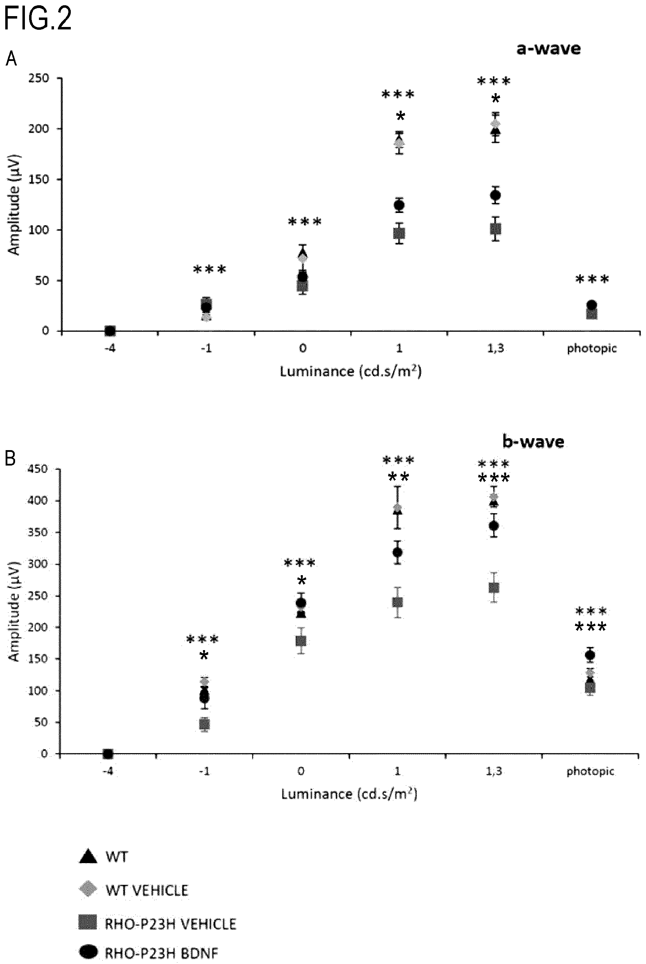

**Figure 2** shows ERG a-wave amplitude (panel a), and b-wave amplitude (panel b) responses recorded at P60 in WT untreated mice (WT), WT mice treated with vehicle (WT VEHICLE), RHO-P23H mice treated with vehicle (RHO-P23H VEHICLE), and RHO-P23H mice treated with a single injection of rhBDNF (RHO-P23H BDNF), according to Example 1. Error bars represent SEM; statistical significance is indicated with asterisks (unpaired Student's t test, Vehicle vs WT (six-pointed asterisks) , and rhBDNF vs Vehicle (five-pointed asterisks)) (*p≤ 0.05, **p≤ 0.01, ***p≤ 0.001; *p≤ 0.05, **p≤ 0.01, ***p ≤ 0.001).

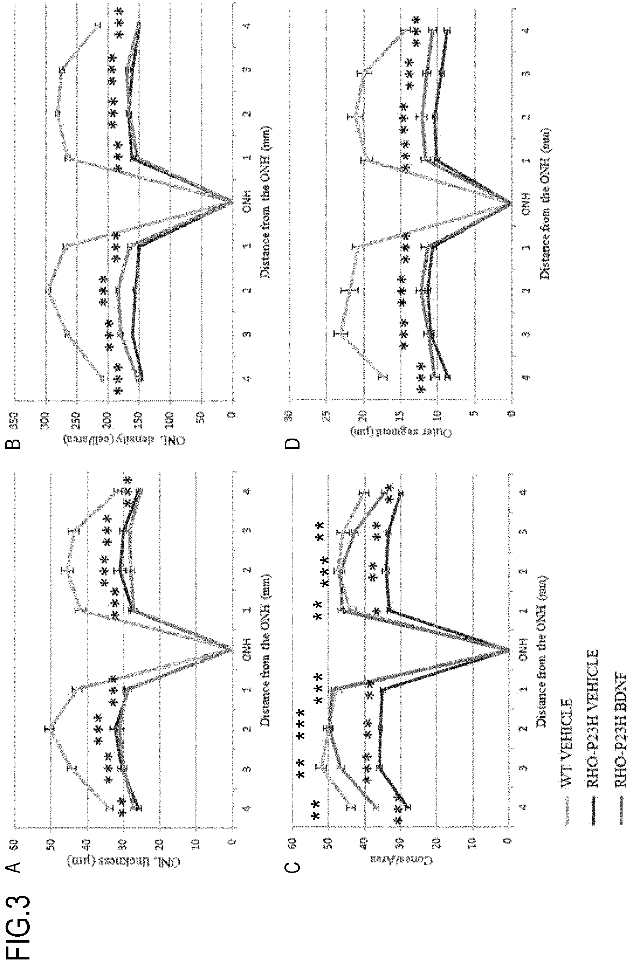

**Figure 3** shows ONL thickness expressed in µm (panel a), ONL density expressed as cell/area (panel b), Cones/Area (number of cones/0,006mm² in the ONL) (panel c), OS thickness expressed in µm (panel d) in vehicle-treated WT (WT VECHICLE), rhBDNF-treated RHO-P23H (RHO-P23H BDNF) and vehicle-treated RHO-P23H (RHO-P23H VEHICLE) retinae, according to Example 1. Statistical significance is indicated with asterisks (unpaired Student's t test, Vehicle vs WT (six-pointed asterisks), and rhBDNF vs Vehicle (five-pointed asterisks)) (*p≤ 0.05, **p≤ 0.01, ***p≤ 0.001; *p≤ 0.05, **p≤ 0.01, ***p ≤ 0.001).

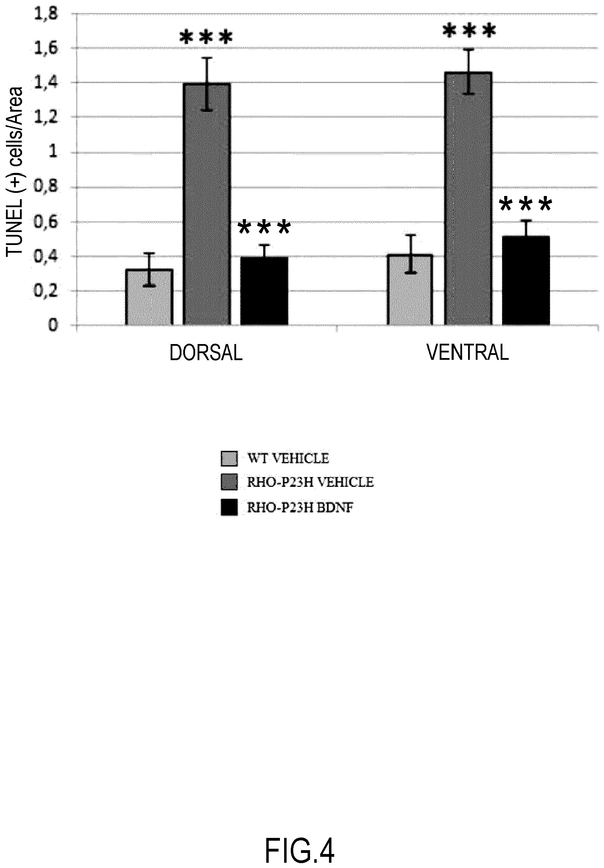

**Figure 4** shows a bar graph representing the number of TUNEL positive cells/0.006mm² in the ONL from vehicle-treated WT (WT VEHICLE), rhBDNF-treated RHO-P23H (RHO-P23H BDNF) and vehicle-treated RHO-P23H (RHO-P23H VEHICLE) mice retinae, according to Example 1. Statistical significance is indicated with asterisks (unpaired Student's t test, Vehicle vs WT (six-pointed asterisks), and rhBDNF vs Vehicle (five-pointed asterisks)) (***p≤ 0.001; ***p ≤ 0.001).

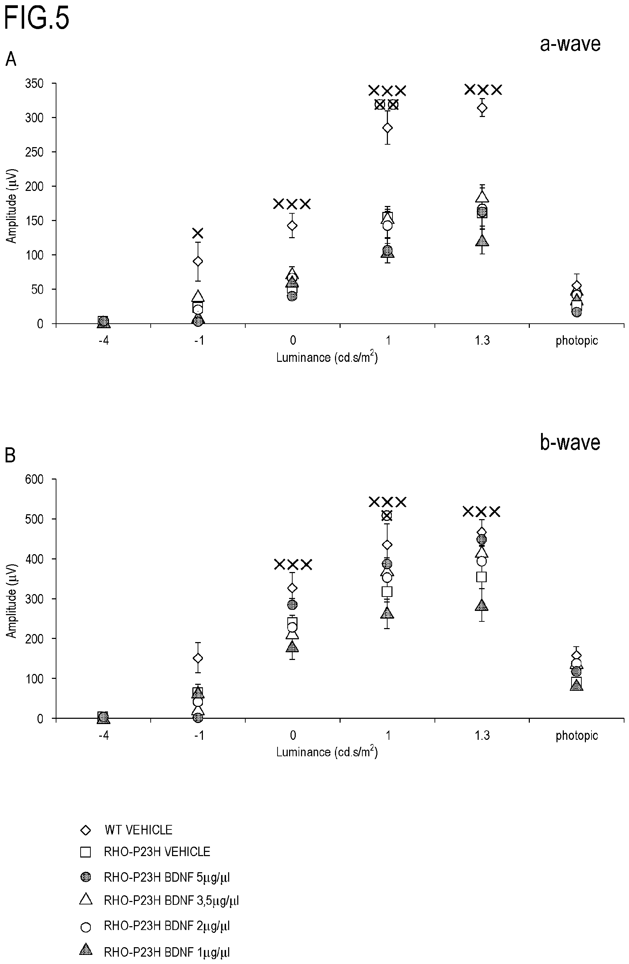

**Figure 5****:** panels (a) and (b) respectively show ERG a-wave amplitude (panel a) and b-wave (panel b) amplitude responses recorded at P30 of vehicle-treated WT mice (WT VECHICLE), vehicle-treated RHO-P23H mice (RHO-P23H VEHICLE), RHO-P23H mice treated with 5µg/µl BDNF (RHO-P23H BDNF 5µg/µl), RHO-P23H treated with 3.5µg/µl BDNF (RHO-P23H BDNF 3.5µg/µl), RHO-P23H treated with 2µg/µl BDNF (RHO-P23H BDNF 2µg/µl), and RHO-P23H treated with 1 µg/µl BDNF (RHO-P23H BDNF 1 µg/µl), injected at PN18, according to Example 2. Error bars represent SEM. Statistical significance is indicated with asterisks (unpaired Student's t test, Vehicle vs WT (× asterisks); rhBDNF 5µg/µl vs Vehicle ( 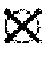 asterisks); rhBDNF 3.5µg/µl vs Vehicle (*asterisks); rhBDNF 2µg/µl vs Vehicle ( 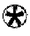 asterisks); rhBDNF 1µg/µl vs Vehicle ( 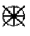 asterisks)) (*p≤ 0.05, **p≤ 0.01, ***p≤ 0.001; these p values are also valid for the other asterisks).

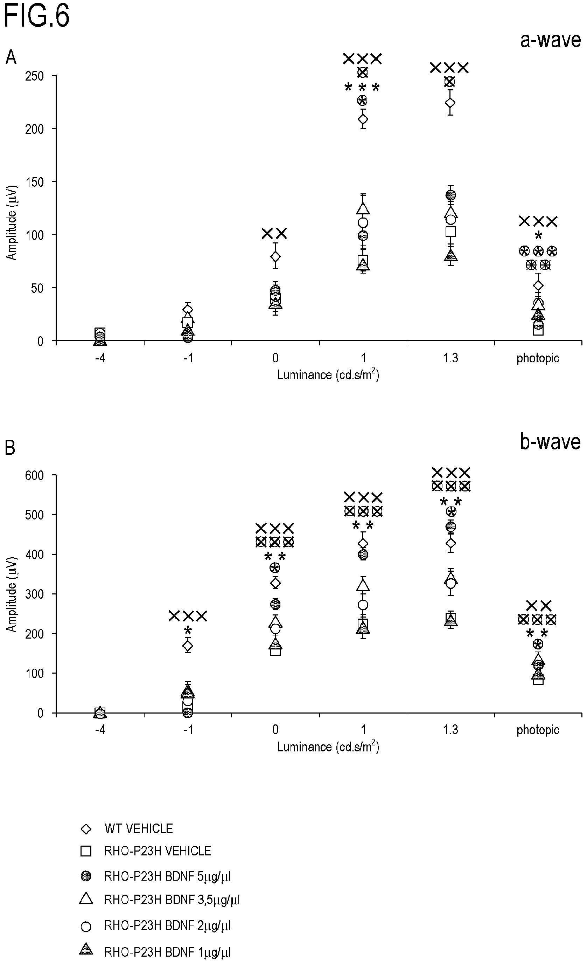

**Figure 6****:** panels (a) and (b) respectively show ERG a-wave amplitude (panel a) and b-wave (panel b) amplitude responses recorded at P60 of vehicle-treated WT mice (WT VECHICLE), vehicle-treated RHO-P23H mice (RHO-P23H VEHICLE), RHO-P23H mice treated with 5µg/µl BDNF (RHO-P23H BDNF 5µg/µl), RHO-P23H treated with 3.5µg/µl BDNF (RHO-P23H BDNF 3.5µg/µl), RHO-P23H treated with 2µg/µl BDNF (RHO-P23H BDNF 2µg/µl), and RHO-P23H treated with 1 µg/µl BDNF (RHO-P23H BDNF 1 µg/µl), injected at PN18, according to Example 2. Error bars represent SEM. Statistical significance is indicated with asterisks (unpaired Student's t test, Vehicle vs WT (× asterisks); rhBDNF 5µg/µl vs Vehicle ( 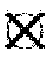 asterisks); rhBDNF 3.5µg/µl vs Vehicle (*asterisks); rhBDNF 2µg/µl vs Vehicle ( 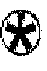 asterisks); rhBDNF 1µg/µl vs Vehicle ( 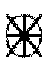 asterisks)) (*p≤ 0.05, **p≤ 0.01, ***p≤ 0.001; these p values are also valid for the other asterisks).

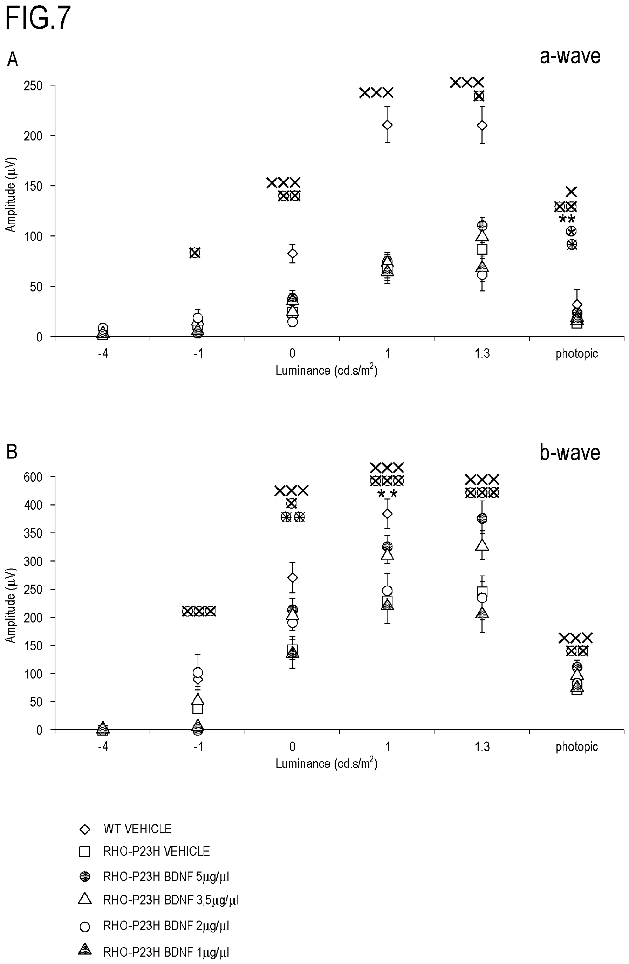

**Figure 7****:** panels (a) and (b) respectively show ERG a-wave amplitude (panel a) and b-wave (panel b) amplitude responses recorded at P90 of vehicle-treated WT mice (WT VECHICLE), vehicle-treated RHO-P23H mice (RHO-P23H VEHICLE), RHO-P23H mice treated with 5µg/µl BDNF (RHO-P23H BDNF 5µg/µl), RHO-P23H treated with 3.5µg/µl BDNF (RHO-P23H BDNF 3.5µg/µl), RHO-P23H treated with 2µg/µl BDNF (RHO-P23H BDNF 2µg/µl), and RHO-P23H treated with 1 µg/µl BDNF (RHO-P23H BDNF 1 µg/µl), injected at PN18, according to Example 2. Error bars represent SEM. Statistical significance is indicated with asterisks (unpaired Student's t test, Vehicle vs WT (× asterisks); rhBDNF 5µg/µl vs Vehicle ( 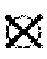 asterisks); rhBDNF 3.5µg/µl vs Vehicle (*asterisks); rhBDNF 2µg/µl vs Vehicle ( 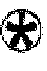 asterisks); rhBDNF 1µg/µl vs Vehicle ( 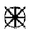 asterisks)) (*p≤ 0.05, **p≤ 0.01, ***p≤ 0.001; these p values are also valid for the other asterisks).

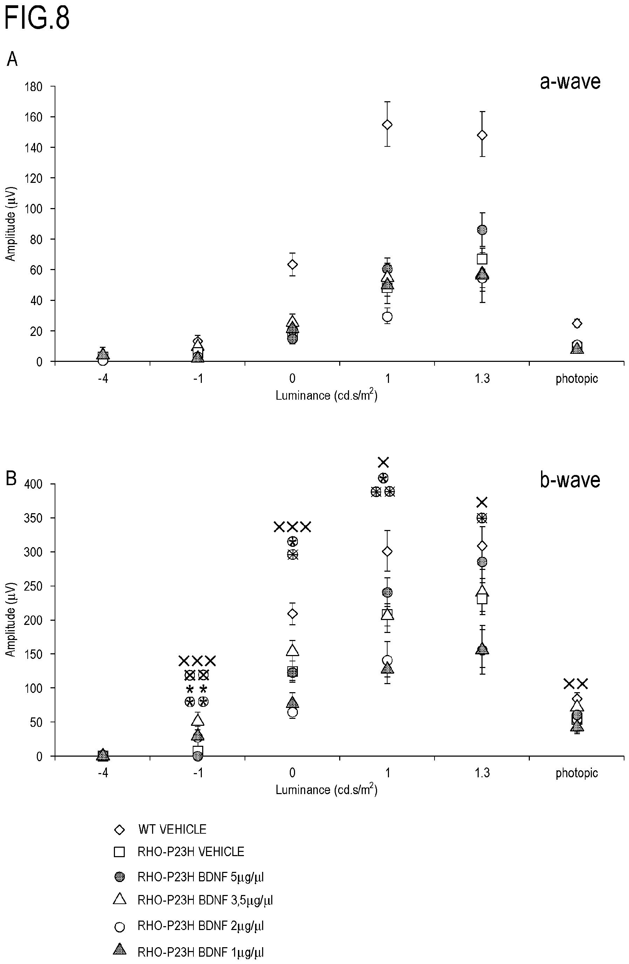

**Figure 8****:** panels (a) and (b) respectively show ERG a-wave amplitude (panel a) and b-wave (panel b) amplitude responses recorded at P120 of vehicle-treated WT mice (WT VECHICLE), vehicle-treated RHO-P23H mice (RHO-P23H VEHICLE), RHO-P23H mice treated with 5µg/µl BDNF (RHO-P23H BDNF 5µg/µl), RHO-P23H treated with 3.5µg/µl BDNF (RHO-P23H BDNF 3.5µg/µl), RHO-P23H treated with 2µg/µl BDNF (RHO-P23H BDNF 2µg/µl), and RHO-P23H treated with 1 µg/µl BDNF (RHO-P23H BDNF 1 µg/µl), injected at PN18, according to Example 2. Error bars represent SEM. Statistical significance is indicated with asterisks (unpaired Student's t test, Vehicle vs WT (× asterisks); rhBDNF 5µg/µl vs Vehicle ( 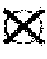 asterisks); rhBDNF 3.5µg/µl vs Vehicle (*asterisks); rhBDNF 2µg/µl vs Vehicle ( 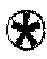 asterisks); rhBDNF 1µg/µl vs Vehicle ( 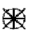 asterisks)) (*p≤ 0.05, **p≤ 0.01, *** p≤ 0.001; these p values are also valid for the other asterisks).

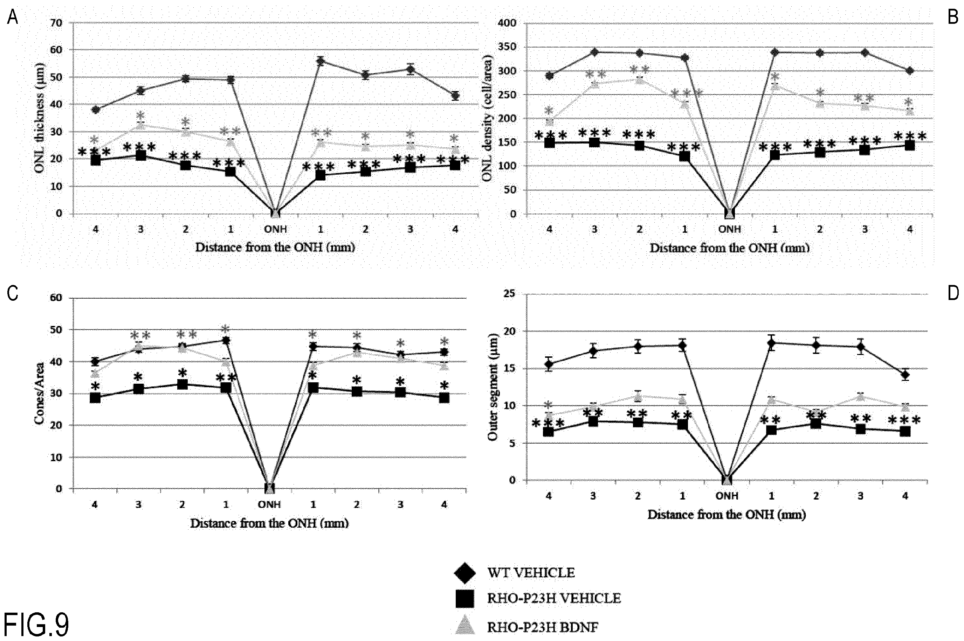

**Figure 9** shows ONL thickness expressed in µm (panel a), ONL density expressed as cell/area (panel b), Cones/Area (number of cones/0,006mm² in the ONL) (panel c), OS thickness expressed in µm (panel d) in vehicle-treated WT (WT VECHICLE), rhBDNF-treated RHO-P23H (RHO-P23H BDNF) and vehicle-treated RHO-P23H (RHO-P23H VEHICLE) retinae, according to Example 2. Statistical significance is indicated with asterisks (unpaired Student's t test, Vehicle vs WT (black asterisks), and rhBDNF vs Vehicle (light grey asterisks)) (*p≤ 0.05, **p≤ 0.01, ***p≤ 0.001).

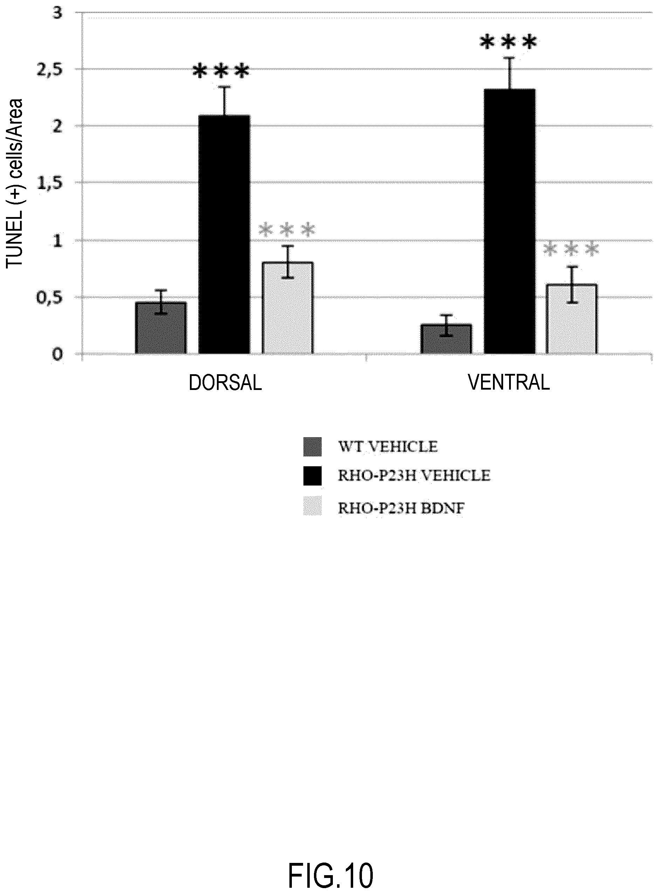

**Figure 10** shows a bar graph representing the number of TUNEL positive cells/0.006mm² in the ONL from vehicle-treated WT (WT VEHICLE), rhBDNF-treated RHO-P23H (RHO-P23H BDNF) and vehicle-treated RHO-P23H (RHO-P23H VEHICLE) mice retinae, according to Example 2. Statistical significance is indicated with asterisks (unpaired Student's t test, Vehicle vs WT (black asterisks), and rhBDNF vs Vehicle (light grey asterisks)) (***p≤ 0.001).

### DETAILED DESCRIPTION OF THE INVENTION

A first object of the invention is brain-derived nerve factor (BDNF) for use in the treatment of rhodopsin-mediated retinitis pigmentosa in a subject.

In the context of a degenerative and progressive disease such as retinitis pigmentosa, the term "treatment", as used herein, refers to the amelioration of the disease or of one or more of the symptoms associated thereof or to the halting or slowing of the progression of the disease. Preferably, said subject is a human subject.

Preferably, said rhodopsin-mediated retinitis pigmentosa is a rhodopsin-mediated autosomal dominant retinitis pigmentosa.

More preferably, said rhodopsin-mediated autosomal dominant retinitis pigmentosa is a rhodopsin-mediated autosomal dominant retinitis pigmentosa associated with a class I rhodopsin mutation. Preferably, said class I rhodopsin mutation is selected from one of the following mutations:
L328P, T342M, Q344R, Q344P, Q344ter, V345L, V345M, A346P, P347A, P347R, P347Q, P347L, P347S, P347T, ter349Q, ter349E.

More preferably, said rhodopsin-mediated autosomal dominant retinitis pigmentosa is a rhodopsin-mediated autosomal dominant retinitis pigmentosa associated with a class II rhodopsin mutation.

Preferably, said class II rhodopsin mutation is selected from one of the following mutations:
N15S, T17M, V20G, P23A, P23H, P23L, Q28H, G51R, G51V, P53R, T58R, T58M, V87D, G89D, G106R, G106W, C110F, C110R, C110S, C110Y, E113K, L125R, W161R, A164E, A164V, C167R, C167W, P171Q, P171L, P171S, Y178N, Y178D, Y178C, E181K, G182S, G182V, C185R, C187G, C187Y, G188R, G188E, D190N, D190G, D190Y, H211R, H211P, C222R, P267R, P267L, S270R, K296N, K296E and K296M.

More preferably, said rhodopsin-mediated retinitis pigmentosa is a rhodopsin-mediated autosomal dominant retinitis pigmentosa associated with a P23H rhodopsin mutation.

Preferably, said rhodopsin-mediated retinitis pigmentosa is in the early or mid-stage.

Preferably, said BDNF is human BDNF, more preferably it is recombinant human BDNF (rhBDNF). Preferably, said BDNF for use as above described is administered to said subject by intravitreal injection.

Preferably, said BDNF is administered to said subject by intravitreal injection in an amount/eye per each administration between 20 µg and 500 µg, preferably between 50 µg and 400 µg, more preferably between 100 µg and 300 µg, even more preferably of 150, 175 or 250 µg.

The administration schedule may vary depending on the patient and it is selected by the skilled person based on the characteristics of the subject to be treated, the severity of the disease and the tests carried out during the treatment to monitor response and progression of disease. Preferably, said administration to said subject by intravitreal injection is repeated once every two years, more preferably once every six months, even more preferably once every three or four months.

According to a preferred schedule of administration, at the beginning of treatment two or three first intravitreal injections are given every three or four months, and the subsequent injections are given six months to two years apart, more preferably six months to one year apart.

A further object of the present invention is an ophthalmic composition suitable for intravitreal administration comprising said BDNF and at least one ophthalmically acceptable excipient. Preferably, said composition is administered to said subject by intravitreal injection.

By "ophthalmically acceptable excipient" as used herein, it is meant an excipient which is suitable for administration to the eye of a subject.

Preferably, said ophthalmically acceptable excipient is selected from solvents, thickening agents, mucoadhesive agents, buffers, antioxidants and preservatives.

A further object of the present invention relates to the above ophthalmic composition for use in the treatment of rhodopsin-mediated retinitis pigmentosa in a subject.

Preferably, said rhodopsin-mediated retinitis pigmentosa is a rhodopsin-mediated autosomal dominant retinitis pigmentosa, more preferably a rhodopsin-mediated autosomal dominant retinitis pigmentosa associated with a class I or II rhodopsin mutation, even more preferably a rhodopsin-mediated autosomal dominant retinitis pigmentosa associated with a class II rhodopsin mutation.

Said class I or class II rhodopsin mutations are preferably selected from the mutations reported above.

More preferably, said rhodopsin-mediated retinitis pigmentosa is a rhodopsin-mediated autosomal dominant retinitis pigmentosa associated with a P23H rhodopsin mutation.

Preferably, said ophthalmic composition is an ophthalmic liquid composition.

Said ophthalmic liquid composition may be in form of a solution or suspension, preferably it is a solution.

Preferably, said ophthalmic liquid composition contains a physiological saline solution as a major vehicle.

Preferably, the pH of said ophthalmic liquid composition is between 4.5 and 8.0, preferably around 7.

Preferably, the concentration of BDNF in said ophthalmic liquid composition is between 0.4 µg/µl and 10 µg/µl, preferably between 1 µg/µl and 8 µg/µl, more preferably between 2 µg/µl and 6 µg/µl, even more preferably selected from 3, 3.5 or 5 µg/µl.

The ophthalmic liquid composition according to the invention may be suitably formulated using appropriate methods known in the art or by the method disclosed in Remington's Pharmaceutical Science (recent edition), Mack Publishing Company, Easton Pa.

In a further aspect, the present invention relates to a method for the treatment of rhodopsin-mediated retinitis pigmentosa, as above described, in a subject in need thereof, comprising administering to the subject BDNF, as described above, in a therapeutically effective amount by intravitreal injection.

Preferably, said method comprises a step of diagnosing the subject for presence of a rhodopsin-mediated retinitis pigmentosa and administering to the subject an effective amount BDNF, as described above, by intravitreal injection. Preferably, said rhodopsin-mediated retinitis pigmentosa is a rhodopsin-mediated autosomal dominant retinitis pigmentosa.

More preferably, said rhodopsin-mediated autosomal dominant retinitis pigmentosa is a rhodopsin-mediated autosomal dominant retinitis pigmentosa associated with a class I or class II rhodopsin mutation.

Preferably, said class I rhodopsin mutation is selected from one of the following mutations:
L328P, T342M, Q344R, Q344P, Q344ter, V345L, V345M, A346P, P347A, P347R, P347Q, P347L, P347S, P347T, ter349Q, ter349E.

Preferably, said method comprises administering BDNF formulated in the above-described ophthalmic composition.

More preferably, said rhodopsin-mediated autosomal dominant retinitis pigmentosa is a rhodopsin-mediated autosomal dominant retinitis pigmentosa associated with a class I or II rhodopsin mutation preferably class II.

Said class I or class II rhodopsin mutations are preferably selected from the mutations reported above.

More preferably, said rhodopsin-mediated retinitis pigmentosa is a rhodopsin-mediated autosomal dominant retinitis pigmentosa associated with a P23H rhodopsin mutation.

Preferably, in combination with the above preferred embodiments, said rhodopsin-mediated retinitis pigmentosa is in the early or mid-stage.

The invention will be further described in the following examples, which do not limit the scope of the invention defined in the claims.

### EXPERIMENTAL SECTION

### Example 1

The pharmacological efficacy in rescuing retinal degeneration of a single intravitreal injection of rhBDNF (3.5µg/µl) on eyes of heterozygous RHO-P23H knock-in mice (RHO-P23H mice) was tested. The tested animal groups were the following:
- a group of 10 RHO-P23H mice received an intravitreal injection of 1µl of a solution containing 3.5µg/µl rhBDNF at post-natal ("PN" or "P") 18 days in both eyes (RHO-P23H BDNF);
- as RHO-P23H controls, 10 RHO-P23H mice received an intravitreal injection of 1µl of vehicle at PN18 days in both eyes (RHO-P23H VEHICLE).

As WT (wild-type) control, WT animals were analyzed following these procedures:
- a group of 6 WT mice received an intravitreal injection of 1µl of vehicle at P18 days in both eyes (WT VEHICLE);
- a group of 6 untreated (un-injected) WT mice were used as control animals to calibrate the instruments and evaluate the ERG in physiological conditions (WT).

In order to perform intravitreal administrations, anesthesia was performed with an intraperitoneal injection of Ketamine/xylazine, and the body temperature monitored with a rectal thermometer and kept constantly at 37.5°C; then, intravitreal administrations of rhBDNF solution (1µl at 3.5µg/µl BDNF) or vehicle (50 mM sodium phosphate monobasic monohydrate, 100 mM sodium chloride, pH 7.2), according to the experimental groups, were carried out by Hamilton series 7000 borosilicate glass syringe not siliconized on both eyes of mice.

The above-mentioned rhBDNF solution was according to the following: 3.5µg/µl BDNF, 50 mM sodium phosphate monobasic monohydrate, 100 mM sodium chloride, pH 7.2.

To evaluate the photoreceptors' function both Scotopic and Photopic standard electroretinogram (ERG) analyses were performed on said animals at P30 and P60 days, respectively.

The electrophysiological analyses were performed by using the following procedures: the animals were adapted to the dark for a period of time of 16 hours and, after anesthesia carried out by an intraperitoneal injection of Ketamine/xylazine, the pupils were dilated with 1% tropicamide (Visufarma, Rome, Italy) and the body temperature monitored with a rectal thermometer and kept constantly at 37.5°C.

The electrophysiological signals by both eyes were recorded by two gold electrodes positioned on the cornea. The reference electrodes, on the other hand, were placed under the skin at the frontal region while the ground electrode near the tail. The signal was amplified to 1K gain and filtered from 0.3 to 1KHz. The ERG records to monitor the functional status of retinal photoreceptor/bipolar cells were carried out by monitoring the retina response under 12 flashes of increasing intensity from 10-4 to 20.0 cd.s m⁻² with a temporal frequency of 0.7 Hz. The photopic ERG were performed using a constant background illumination of 50 cd m⁻² and 10 flashes having the same intensity, 20 cd.s m⁻². On average, the exam lasted for 15 minutes.

The results of ERG recorded at P30 are reported in Figure 1A (a-wave), and Figure 1B (b-wave), wherein for the a-wave: 0.0001 cd·s/m2, 6.1 mV; 0.1 cd·s/m2, 52.8 mV; 1 cd·s/m2, 213.4 mV; 10 cd·s/m2, 341.3 mV; 20 cd·s/m2, 401.1 mV; photopic, 26.4 mV; for the b-wave: 0.0001 cd·s/m2, 2.3 mV; 0.1 cd·s/m2, 315.8 mV; 1 cd·s/m2, 506.3 mV; 10 cd·s/m2, 732 mV; 20 cd·s/m2, 876.8 mV; photopic, 188.1 mV.

In particular, while analysis of a-wave amplitude, reflecting the response the cones and rods to light stimuli, did not show any significant difference in the scotopic (rod), mesopic (rod-cone) and photopic (cone) responses in the rhBDNF-treated RHO-P23H mice compared to vehicle-treated RHO-P23H animals (Fig.1A), b-wave amplitude, reflecting the photoreceptor-mediated depolarization of the post-synaptic bipolar cells, revealed a statistically amelioration in photopic response in rhBDNF-treated RHO-P23H mice (Fig.1B).

The results of ERG recorded at P60 are reported in Figure 2A (wave a), and Figure 2B (wave b), wherein for the a-wave: 0.0001 cd·s/m², 6.1 mV; 0.1 cd·s/m², 52.8 mV; 1 cd·s/m², 213.4 mV; 10 cd·s/m², 341.3 mV; 20 cd·s/m², 401.1 mV; photopic, 26.4 mV; for the b-wave: 0.0001 cd·s/m², 2.3 mV; 0.1 cd·s/m², 315.8 mV; 1 cd·s/m², 506.3 mV; 10 cd·s/m², 732 mV; 20 cd·s/m², 876.8 mV; photopic, 188.1 mV.

Notably, at P60, 42 days post-injections, the ERG profiles of rhBDNF treated RHO-P23H mice showed a stop of the progressive retinal degeneration compared to controls.

In particular, analysis of a-wave amplitude revealed a statistically amelioration in both mesopic and photopic responses in the rhBDNF-treated RHO-P23H mice (RHO-P23H BDNF) compared to vehicle-treated RHO-P23H mice (RHO-P23H VEHICLE) (Fig. 2A). Similar results were obtained when b-wave amplitude was analyzed: analysis of b-wave amplitude indeed revealed a statistically amelioration in all of scotopic, mesopic and photopic responses to light stimuli in the rhBDNF-treated RHO-P23H mice compared to vehicle-treated RHO-P23H animals (Fig. 2B).

Thus, the obtained data shows that a single intravitreal administration of rhBDNF (3.5µg/µl) attenuates photoreceptors' degeneration, and recovers visual function in RHO-P23H knock-in mice after 42 days from injection. The improved ERG responses are mainly attributable to a better-preserved photoreceptors' function, and to an attenuation of the degeneration.

The morphological, phenotypic effect of rhBDNF administration RHO-P23H knock-in mice was also investigated at P60.

In particular, said morphological and phenotypic effect of rhBDNF administration was assessed on cryosections obtained by the above-mentioned animal groups, excluding WT untreated animal group, by evaluating: the length (i.e. the thickness) of the outer segment of (OS) of rods, the length (i.e. the thickness) and density of the outer nuclear layer (ONL), as well as the structure and number of cone cells.

Moreover, to quantify the protective effect of rhBDNF treatment on retinal degeneration, the level of apoptosis in the retinal cryosection was evaluated by TdT-mediated dUTP nick end labelling (TUNEL) assay, which identifies the nuclei containing fragmented DNA (i.e. apoptotic nuclei) in each entire retina section.

The above-mentioned retinal cryosections were obtained according to the following. RPE and Retina tissues from a single eye for each treated animal above were dissected and fixed overnight in 4% paraformaldehyde in PBS at 4°C, then cryopreserved by treatment first with 15%, and then with 30% sucrose in phosphate-buffered saline and embedded in OCT. 20µm cryosections were collected on slides (Superfrost Plus; Fisher Scientific, Pittsburgh, PA).

The evaluation of number and structure of cone cells in said retinal cryosections was performed by counting the cells positive for cone-Arrestin immunofluorescence in standard areas at a comparable distance from the optic nerve.

The cone-Arrestin immunofluorescence protocol was carried out according to the following.

Permeabilization of said retinal cryosections was performed with NP40 1% for 15 minutes at room temperature. Retinae were rinsed 3 times in PBS/0.1% Tween-20 for 30 minutes, and blocked for 1 hour at room temperature (RT) in blocking solution (10% FBS in PBS/0.1% Tween-20). The thus-obtained (thawed) retinae were then incubated in primary antibody in blocking solution overnight at 4 °C on a rocking platform. The next day, retinae were rinsed 3 times in PBS/0.1% Tween-20 for 30 minutes and placed in secondary antibody in 5% FBS in PBS/0.1% Tween-20 1 hour at RT. Retinae were rinsed 3 times in PBS for 30 minutes, the slides were counterstained with 4,6-diamidino-2-phenylindol, DAPI (Vector Laboratories H-1200) and then flattened between two coverslips for confocal imaging. The following primary antibodies were used: mouse anti-Rhodopsin (1:100, Abcam ab5417), rabbit anti-Cone-Arrestin (1:1000, EMD Millipore AB15282). Secondary antibodies were conjugated to Alexa 488 goat anti-rabbit/mouse (1:1000, Invitrogen A-11008, A-11001). Coverslips were mounted onto glass slides with 70% glycerol in PBS or dehydrated before mounting (Eukitt Mounting Medium; EMS, Fort Washington, PA). Slides were imaged using the LSM710 Zeiss Confocal Microscopy system. All samples were imaged, and images were identically processed. ImageJ software was used for processing images, and the average number of photoreceptor cells per 200 µm of retina was calculated. The number of cones/area was evaluated by manual counts with a Leica DM-6000 microscope, with the objective Leica ∞/0.17/D, HCX PL FLUOTAR, 40X/0.75 that has an area of 0.31 mm².

The same retinal sections used for cone counts were imaged using a Leica TCS SPE 40 X confocal microscope acquiring Z-stacks with the DAPI wavelength. The images were used to calculate the number of ONL nuclei (ONL cell density) and ONL thickness. The evaluation of the number of ONL nuclei was performed by counting the DAPI-stained nuclei in areas at a comparable distance from the optic nerve. The selection of each area and the nuclei counting were done manually using the ITEM Analysis Image Processing program that stores and then processes the results. ONL was be measured manually close to the optic nerve head in both the superior and inferior retina using the ITEM Analysis Image Processing program.

Evaluation of the Outer Segment (OS) of rods was performed by measuring the length (i.e. thickness) of OS of the cells positive for anti-Rhodopsin staining in standard areas, at a comparable distance from the optic nerve with a Leica DM-6000 microscope.

The results obtained are shown in Figures 3A-D.

In particular, the rhBDNF-treated RHO-P23H (RHO-P23H BDNF) retinae showed a slight increase in the length of OS of rods (OS thickness, Fig.3D), compared to vehicle-treated RHO-P23H (RHO-P23H VEHICLE) eyes on both ventral and dorsal sides, while no change in outer nuclear layer (ONL) thickness (i.e. the length of ONL, Fig. 3A) and density (i.e. the density of nuclei within the ONL, Fig. 3B) was seen on either side of the RHO-P23H retina by treatment of rhBDNF, suggesting rhBDNF may not be sufficient to protect the RHOP23H rods from the fast period of retinal degeneration. Surprisingly, a significant recovery of cone number defects in rhBDNF-treated RHO-P23H retinas compared to control vehicle-treated RHO-P23H mice was observed, as demonstrated by quantifying cones/area (Fig. 3C); notably, the surviving cones in the rhBDNF-treated RHO-P23H retinae were more elongated and healthier than in the vehicle-treated RHO-P23H retinae and like vehicle-treated WT.

In agreement with the ERG results, these data demonstrate a slight increase in the number of rods nuclei and length of OS of rods and, most importantly, a significant recovery of cone number in rhBDNF-treated RHO-P23H retinae compared to control vehicle-treated RHO-P23H mice. Specifically, the surviving cones in the rhBDNF-treated RHO-P23H retinas resulted in number and morphology like WT retinas. Notably, this significant rescue in cones was consistent with the observed rescue in retinal function by ERG analysis of Example 1.

Regarding the assessment of the extent and distribution of apoptotic cell death in the above-mentioned retinal cryosections, the above TUNEL assay was carried out using the In Situ Cell Death Detection Kit, POD (Roche 11684817910) following the manufacturer's directions. As negative control to evaluate possible unspecific results, fixed and permeabilized retina sections were incubated with the reaction mix without TUNEL reaction enzyme. Sections were observed with a Leica DM-6000 microscope and then confocal images were acquired using the LSM710 Zeiss Confocal Microscopy system.

The results of the TUNEL assay are reported in Figure 4.

In particular, the rhBDNF treatment induced a significant decrease in TUNEL-positive cells in rhBDNF-treated RHO-P23H mice, compared with vehicle-treated RHO-P23H mice; surprisingly, the number of TUNEL-positive cells in the rhBDNF-treated RHO-P23H retinas was substantially similar to those of the vehicle-treated WT-control group.

The TUNEL assay results showed therefore that rhBDNF administration reduces the number of apoptotic cells in the retina of the RHO-P23H mice, thus supporting the therapeutic efficacy of rhBDNF administration.

Altogether, the obtained results support the conclusion that therapeutic administration of rhBDNF results in morphological and functional rescue of retinal degeneration, characterized by a delay of rods' degeneration, in a block of the secondary cone cell death, and in functional rescue of photoreceptor function.

### Example 2

The dose-response of rhBDNF intravitreal (IVT) administration on heterozygous RHO-P23H knock-in mice (RHO-P23H mice) in blocking the retinal degeneration was evaluated, and the duration of the beneficial effect of the rhBDNF treatment was assessed.

The animals were divided in the following experimental groups:
- Group 1 (G1, WT vehicle): 6 WT animals received an intravitreal injection of 1µl of vehicle in the left eyes at PN18 (right eyes have been used as no injected control eyes);
- Group 2 (G2, RHO-P23H vehicle): 6 RHO-P23H mice received an intravitreal injection of 1µl of vehicle in the left eyes at PN18 (right eyes have been used as no injected control eyes);
- Group 3 (G3, RHO-P23H BDNF 5 µg/µl): 6 RHO-P23H mice received an intravitreal injection of 1µl of a solution containing rhBDNF at 5µg/µl in both eyes at PN18;
- Group 4 (G4, RHO-P23H BDNF 3.5 µg/µl): 6 RHO-P23H mice received an intravitreal injection of 1µl of a solution containing rhBDNF at 3.5 µg/µl in both eyes at PN18;
- Group 5 (G5, RHO-P23H BDNF 2 µg/µl): 6 RHO-P23H mice received an intravitreal injection of 1µl of a solution containing rhBDNF at 2µg/µl µg/µl in both eyes at PN18;
- Group 6 (G6 RHO-P23H BDNF 1 µg/µl): 6 RHO-P23H mice received an intravitreal injection of 1µl of a solution containing rhBDNF at 1 µg/µl in both eyes at PN18.

The above-mentioned vehicle consisted of 50 mM sodium phosphate monobasic monohydrate, 100 mM sodium chloride, pH 7.2.

The above-mentioned rhBDNF solutions were according to the following: BDNF at the concentration specific for each above-mentioned animal group, 50 mM sodium phosphate monobasic monohydrate, 100 mM sodium chloride, pH 7.2.

In order to perform intravitreal administrations, animals were anesthetized as described in Example 1.

Photoreceptor function in each animal group was evaluated by standard electroretinographic (ERG) as described in Example 1, at the following time-points after the administration: PN30 - PN60 - PN90 - PN120.

The ERG results are reported in Figures 5-8, by analyzing scotopic (rod, for the a-wave: 0.0001 cd·s/m2, 6.1 mV; 0.1 cd·s/m2, 52.8 mV; for the b-wave: 0.0001 cd·s/m2, 2.3 mV; 0.1 cd·s/m2, 315.8 mV), mesopic (rod-cone, for the a-wave: 1 cd·s/m2, 213.4 mV; 10 cd·s/m2, 341.3 mV; 20 cd·s/m2, 401.1 mV; for the b-wave: 1 cd·s/m2, 506.3 mV; 10 cd·s/m2, 732 mV; 20 cd·s/m2, 876.8 mV;) and photopic (cone, for the a-wave: 26.4 mV; for the b-wave: 188.1 mV) responses.

At PN30 (i.e., 12 days post-injection), the ERG analysis showed an amelioration in rod and cone responses in both G3 and G4 rhBDNF-treated RHO-P23H mice compared to control mice (G1 and G2) (Figures 5A-5B).

In particular, analysis of b-wave amplitude, reflecting the photoreceptor-mediated depolarization of the post-synaptic bipolar cells, revealed a trend of amelioration in scotopic and mesopic responses in both G3 and G4 rhBDNF-treated RHO-P23H.

Notably, the b-wave amplitude in the G3 rhBDNF-treated RHO-P23H showed a statistically significant increase in both scotopic and mesopic responses. Importantly, light intensities from 0 to 1.3 cd s/m² evoked responses originated in the mesopic range (i.e., by both rod and cone photoreceptors) were similar between G3 and G1 groups.

As in Example 1, the beneficial effect of rhBDNF administration at 5µg/µl was more evident at **P60** (i.e., 42 days post-injection) as shown in Figures 6A-6B. The ERG profiles of both G3 and G4 rhBDNF-treated RHO-P23H mice showed an amelioration of photoreceptors function and a delay in the progression of retinal degeneration compared to controls. Analysis of a-wave amplitude revealed a statistically significant amelioration in mesopic and photopic responses in both G3 and G4 rhBDNF treated RHO-P23H mice compared to vehicle-treated RHO-P23H animals.

Notably, the G3 rhBDNF-treated RHO-P23H mice showed a statistically significant higher amelioration in the mesopic responses compared to the G4 groups.

This result was more evident when b wave amplitude was analyzed. In fact, analysis of b-wave amplitude revealed a statistically significant amelioration in all scotopic, mesopic and photopic responses to light stimuli in both G3 and G4 rhBDNF-treated RHO-P23H mice compared to control animals.

Importantly, rhBDNF at 5µg/µl induced a high statistically significant amelioration of b-wave amplitude in G3 animals, in which mesopic and photopic responses resulted similar to WT mice. Specifically, light intensities from 0 to 1.3 cd s/m2 evoke responses originated in the mesopic range resulted comparable between G3 and G1 groups, suggesting a fully rescue in both rod- and cone-functions.

The amelioration of photoreceptors' function was also evident at PN90 (i.e., 72 days post-injection), as shown in Figures 7A-7B. Analysis of a-wave amplitude of both G3 and G4 rhBDNF treated RHO-P23H mice revealed a slight trend of amelioration in mesopic responses, but a statistically significant amelioration in photopic response.

This result was more evident when b-wave amplitude was analyzed. In fact, analysis of b-wave amplitude revealed a statistically amelioration in all scotopic, mesopic and photopic responses to light stimuli in both G3 and G4 rhBDNF-treated RHO-P23H mice compared to control animals. Again, rhBDNF at 5 µg/µl induced a high statistically significant amelioration of b-wave amplitude in G3 animals, in which mesopic and photopic responses continued to be similar to ERG responses recorded in WT mice. Again, light intensities beyond 1.3 cd s/m² evoke responses originated in the mesopic range comparable between G3 and G1 groups, suggesting a high beneficial effect of the high-dose administration of rhBDNF as detected by rescue in both rod- and cone-functions.

The ERG profiles of G3, but not of G4, rhBDNF-treated RHO-P23H mice still showed a trend of preservation of function compared to controls until **PN120** (i.e., 102 days post-injection), as shown in Figures 8A-8B. Light intensities from 1 to 1.3 cd s/m² evoked responses originated in the mesopic range, that resulted in a positive trend of increase in G3 rhBDNF-treated RHOP23H mice compared to controls. This result suggests a maintenance of beneficial effect of rhBDNF at 5 µg/µl in both rod- and cone-functions after about 4 months post-injection. In contrast, the administration of the rhBDNF at 3.5 µg/µl does not show a beneficial effect after about 4 months post-injection. However, a residue of amelioration is yet appreciable at light intensities from -1 to 0 cd s/m².

Regarding G5 rhBDNF-treated RHO-P23H mice, at PN30 and PN60, the ERG analysis showed an amelioration in rod and cone responses in rhBDNF-treated RHO-P23H mice similar to that observed in the G4 rhBDNF-treated RHO-P23H mice, when a-wave and b-wave were compared to control mice (G1 and G2). In accordance with the beneficial effect of rhBDNF administration, the G5 rhBDNF-treated RHO-P23H mice showed a statistically amelioration in the mesopic responses in both a-wave and b-wave. However, light intensities beyond -1 cd s/m2 always evoke slower responses in G5 compared to those recorded in G4, suggesting that a low-dose of rhBDNF may reduce the beneficial effect of rhBDNF on both rod- and cone-functions (Figures 5 and 6).

The beneficial effect was no longer evident in G5 rhBDNF-treated RHO-P23H mice at PN90 and PN 120. Analysis of both a-wave and b-wave amplitudes, on both PN90 and PN120 old G5 rhBDNF treated RHO-P23H mice, revealed ERG records similar to those recorded in untreated G2 RHO-P23H mice in all scotopic, mesopic and photopic responses to light stimuli. However, similar to G4, a residue of amelioration was yet appreciable when light intensities were from -1 to 0 cd s/m2, suggesting slight maintenance of rod functions after about 4 months post injection (Figures 7 and 8).

As regards the G6 rhBDNF-treated RHO-P23H mice, no beneficial effect was identified at any of the timepoints analyzed (see Figures 5-8).

These data demonstrate the dose-dependent rescue of photoreceptors' function in RHO-P23H knock-in mice, which is greater and more prolonged after a single intravitreal administration of high-dose rhBDNF (5µg/µl).

In particular, the intravitreal administration of rhBDNF at said high dose shows the best prolonged preservation of photoreceptors' function; in contrast, the intravitreal administration of rhBDNF at low dose (1µg/µl) does not show any amelioration of photoreceptors' function.

The morphological, phenotypic effect of rhBDNF administration RHO-P23H knock-in mice was also investigated for the above-mentioned G3 (RHO-P23H mice treated with 5µg/µl rhBDNF) group, in comparison with the above-mentioned G1 (WT vehicle) and G2 (RHO-P23H vehicle) groups, at P120, carrying out the same analyses described in Example 1.

The results of the morphological analysis are reported in Figures 9A-9D.

In particular, a statistically significant improvement of outer nuclear layer (ONL) thickness on either side of optic nerve (i.e. dorsal and ventral sides) of the RHO-P23H retina treated with rhBDNF ("RHO-P23H BDNF"), compared to controls was observed (Fig. 9A). This amelioration was accompanied by a statistically significant improvement of outer nuclear density (Fig. 9B), and an increase in the length of Outer Segment of photoreceptor cells, compared to vehicle-treated RHOP23H eyes, on both ventral and dorsal sides (Fig. 9D), thus demonstrating that said rhBDNF administration at the concentration of 5µg/µl is able to protect the RHOP23H^{+/-} rods from the retinal degeneration. Remarkably, a statistically significant rescue of cone number defects in rhBDNF-treated RHO-P23H retinae, compared to control vehicle-treated RHO-P23H mice, was also observed (Fig. 9C); in particular, both number and structure of the surviving cones was surprisingly comparable to those of vehicle-treated WT retina. This significant rescue in cones was consistent with the observed rescue in retinal function shown by G3 group ERG analysis.

Altogether these data support that rhBDNF administration at the concentration of 5µg/µl fully rescues cones' cell death in RHO-P23H mice.

Consistent with the above-mentioned morphological results, treatment with rhBDNF at the concentration of 5µg/µl resulted in a significant decreased number of TUNEL-positive photoreceptor cells compared to vehicle-treated RHO-P23H mice (Fig. 10), supporting a reduction in photoreceptors' cell death.

In fact, rhBDNF treatment induced a statistically significant decrease in TUNEL-positive cells in rhBDNF-treated RHO-P23H mice, (compared with vehicle-treated RHO-P23H mice) being indeed highly similar than the vehicle-treated control group. These data strongly confirm the effectiveness of rhBDNF treatment in rescuing apoptotic events in the retina of the RHO-P23H mice.

According to the results shown above, rhBDNF treatment at the concentration of 5µg/µl led to an improvement in functionality of photoreceptors, which is accompanied by the rescue of retinal cell death and morphology up to 4 months of age (i.e. 102 days after injection). Notably, the effectiveness of the treatment was evident in the statistically significant amelioration of ONL thickness and density, as well as the fully restoration of functionality and survival of cones, which were numerically and morphologically substantially identical to those of the control vehicle-treated WT retina.

## Claims

1. Brain-derived nerve factor (BDNF) for use in the treatment of rhodopsin-mediated retinitis pigmentosa in a subject.

2. BDNF for use as claimed in claim 1, wherein said rhodopsin-mediated retinitis pigmentosa is a rhodopsin-mediated autosomal dominant retinitis pigmentosa.

3. BDNF for use as claimed in claim 2, wherein said rhodopsin-mediated autosomal dominant retinitis pigmentosa is associated with a class I rhodopsin mutation.

4. BDNF for use as claimed in claim 3, wherein said class I rhodopsin mutation is selected from one of the following mutations:
L328P, T342M, Q344R, Q344P, Q344ter, V345L, V345M, A346P, P347A, P347R, P347Q, P347L, P347S, P347T, ter349Q, ter349E.

5. BDNF for use as claimed in claim 2, wherein said rhodopsin-mediated autosomal dominant retinitis pigmentosa is associated with a class II rhodopsin mutation.

6. BDNF for use as claimed in claim 5, wherein said class II rhodopsin mutation is selected from one of the following mutations:
N15S, T17M, V20G, P23A, P23H, P23L, Q28H, G51R, G51V, P53R, T58R, T58M, V87D, G89D, G106R, G106W, C110F, C110R, C110S, C110Y, E113K, L125R, W161R, A164E, A164V, C167R, C167W, P171Q, P171L, P171S, Y178N, Y178D, Y178C, E181K, G182S, G182V, C185R, C187G, C187Y, G188R, G188E, D190N, D190G, D190Y, H211R, H211P, C222R, P267R, P267L, S270R, K296N, K296E and K296M.

7. BDNF for use as claimed in claim 5, wherein said class II rhodopsin mutation is P23H.

8. BDNF for use as claimed in claims 1 to 7, wherein said BDNF is administered to said subject by intravitreal injection.

9. BDNF for use as claimed in claims 1 to 8, wherein said BDNF is human BDNF, preferably it is recombinant human BDNF.

10. BDNF for use as claimed in claims 1 to 9, wherein said BDNF is administered to said subject by intravitreal injection in an amount/eye per each administration between 20 µg and 500 µg, preferably between 50 µg and 400 µg, more preferably between 100 µg and 300 µg, even more preferably selected from 150, 175 or 250 µg.

11. An ophthalmic composition comprising BDNF and at least one ophthalmically acceptable excipient, for use in the treatment of rhodopsin-mediated retinitis pigmentosa in a subject.

12. An ophthalmic composition for use as claimed in claim 11, wherein said BDNF is human BDNF, more preferably it is recombinant human BDNF.

13. An ophthalmic composition for use as claimed in claim 11 or 12, which is a liquid composition.

14. An ophthalmic composition for use as claimed in claim 13, wherein BDNF is contained in the composition at a concentration between 0.4 µg/µl and 10 µg/µl, preferably between 1 µg/µl and 8 µg/µl, more preferably between 2 µg/µl and 6 µg/µl, even more preferably selected from 3, 3.5 or 5 µg/µl.
